# FASCICULE DE BREVET EUROPEEN

(11) **EP 2 736 468 B1**
(45) Date de publication et mention de la délivrance du brevet: **27.04.2016**
(21) Numéro de dépôt: 12744102.0
(22) Date de dépôt: 24.07.2012
(51) Int. Cl.: A61F 11/04, A61F 11/00, H04R 25/00, A61N 7/00, A61N 1/375, A61B 8/08, A61N 1/05, A61N 1/36, A61B 8/00

(54) **DISPOSITIF DE TRAITEMENT DE LA CAPACITE SENSORIELLE D'UNE PERSONNE**
VORRICHTUNG ZUR BEHANDLUNG DER SENSORISCHEN KAPAZITÄT EINER PERSON
DEVICE FOR TREATING THE SENSORY CAPACITY OF A PERSON

(30) Priorité: 27.07.2011 FR 1156862; 27.07.2011 US 201161512087 P
(43) Date de publication de la demande: 04.06.2014
(73) Titulaire: Université Pierre et Marie Curie (Paris 6), 75252 Paris Cedex 05 (FR)
(72) Inventeur: CARPENTIER, Alexandre, 75116 Paris (FR)
(74) Mandataire: Thibault, Jean-Marc
(86) Numéro de dépôt international: PCT/FR2012/051757
(87) Numéro de publication internationale: WO 2013/017778

(56) Documents cités:
- WO-A1-2004/052256
- US-A- 4 982 434
- US-B1- 6 584 357

## Description

La présente invention concerne le traitement de la capacité sensorielle d'une personne à l'aide de dispositifs implantables, et elle vise plus précisément, le traitement au sens général des capacités sensorielles d'une personne telles qu'auditive, visuelle, olfactive, tactile voire gustative.

Selon une application préférée, la présente invention concerne un dispositif de traitement de la surdité mettant en oeuvre une émission d'ondes ultrasonores dans le cerveau d'un patient atteint de surdité ou de difficultés d'audition.

Selon une autre application préférée, la présente invention concerne un dispositif de traitement de la vision mettant en oeuvre une émission d'ondes ultrasonores dans le cerveau d'un patient atteint de cécité ou de difficultés visuelles.

Dans le domaine de l'audition, la surdité et les difficultés d'audition relèvent d'une problématique médicale majeure aujourd'hui dans la mesure où ces pathologies ont d'importantes répercussions sur la vie sociale des patients concernés.

De façon générale, on emploie le terme surdité pour des patients atteints d'une perte d'audition profonde, en général supérieure à une perte de 90 dB.

On connaît actuellement trois catégories principales de surdité.

En premier lieu, on connaît la surdité dite « de conduction » qui concerne des pathologies de l'oreille moyenne.

On connaît également la surdité dite« sensorineurale », qui concerne des maladies de la cochlée, située dans l'oreille interne ou des malformations de cette cochlée.

Enfin, on connaît également la surdité dite « neurale », qui découle de pathologies du nerf auditif ou des cortex auditifs du cerveau.

La compréhension des mécanismes physiques et physiologiques de l'audition est particulièrement complexe.

En effet, l'oreille externe collecte les sons et les concentre à l'intérieur de l'oreille externe, sur le tympan. Le tympan transmet ensuite les sons perçus par mise en vibrations de la chaine ossiculaire contenue dans la cavité de l'oreille moyenne. L'oreille moyenne a un rôle d'amplificateur de transmission réalisé par les trois petits os de cette chaine ossiculaire. Le signal sonore amplifié par l'oreille moyenne est ensuite transmis à l'oreille interne remplie de liquide, qui forme la cochlée. L'oreille interne transforme alors le signal en des excitations nerveuses du nerf cochléaire. Pour chaque fréquence sonore, correspond une ou des activations spécifiques de chacune des fibres du nerf cochléaire.

Le nerf cochléaire est dirigé vers le huitième noyau du tronc cérébral et les neurones relaient et conduisent le signal neuronal vers le cortex auditif primaire, dit gyrus de Heschel, au niveau de l'aire 41 de Broadmann, ainsi que l'aire 42 de Broadmann du gyrus temporal planaire (A. Carpentier et al., Neurochirurgie 2002; 48(2-3) : 80-86).

Ensuite, le signal neuronal est traité par les aires auditives secondaires (également appelées « zones associatives ») du cerveau, avant d'atteindre l'aire du langage (Aire 22 de Broadmann, aussi appelée aire de Wernicke).

Dans les cas de difficultés auditives et de surdités, différentes solutions existent aujourd'hui pour permettre à des patients d'être équipés, notamment par des implants cochléaires ou encore des implants du tronc cérébral. Par exemple, plus de 188 000 patients reçoivent annuellement dans le monde des implants cochléaires.

Toutefois, ces implants cochléaires et ces implants du tronc cérébral ne peuvent correctement restaurer l'audition pour plusieurs raisons.

Tout d'abord, ces implants reposent tous sur le concept de stimulation électrique du tissu neuronal. Or une telle stimulation est toujours associée à des phénomènes de diffusion électrique dans le tissu notamment sensorineural. Ainsi, toute stimulation électrique du tissu neuronal adresse *in fine* plusieurs circuits neuronaux sans une spécificité suffisamment maîtrisée.

De plus, les dispositifs de stimulation électrique développés pour les implants cochléaires ou les implants du tronc cérébral comportent tous des électrodes et donc sont hautement invasifs pour le patient, ce qui altère voir même détruit totalement la faible fonction auditive résiduelle naturelle.

Par ailleurs, les systèmes de stimulation électriques nécessitent une implantation dans ou au contact du tissu neuronal, ce qui implique également un risque de positionnement erroné ou non optimal des électrodes.

Enfin, les électrodes de stimulation électrique des implants cochléaires et du tronc cérébral ne peuvent pas respecter fidèlement l'organisation anatomique et fonctionnelle des neurones (somatotopie) dans la mesure où les zones d'implantation sont très denses en neurones, ce qui là encore favorise l'activation de plusieurs circuits neuronaux simultanément.

En conséquence, les patients dotés de tels implants ne perçoivent malheureusement que très peu de sons différents, ce qui ne leur permet pas de suivre correctement ne serait-ce qu'une conversation orale normale d'un niveau sonore habituel.

Afin de résoudre certain de ces problèmes techniques, des recherches ont porté sur le développement de « grilles » électriques, à positionner au contact direct de la surface du lobe temporal du cerveau. Cependant, bon nombre des problèmes précédemment évoqués subsistent.

En effet, lors de la stimulation électrique par ces grilles corticales superficielles, la diffusion au cortex périphérique du cortex auditif primaire et la somatotopie ne peuvent être préservées. De plus, le cortex auditif primaire étant localisé plus profond que la surface du lobe temporal sur laquelle la grille est appliquée, l'excitation électrique produite n'est pas réalisée sur le bon gyrus.

D'autres équipes de recherche ont tenté de résoudre ces problèmes par l'insertion d'électrodes cérébrales. Cependant, cette technique est particulièrement invasive et les mouvements des électrodes, comme des grilles, ne sont pas compatibles avec la pulsatilité physiologique du cerveau à l'intérieur du crâne.

Par ailleurs, il est également connu, par le brevet US 4 982 434 un système de traitement de la surdité destiné à être fixé sur le crâne d'un patient. Ce système comporte un microphone adapté pour capter les sons ambiants. Ce microphone est relié via un convertisseur, à un transducteur d'application de vibrations au crâne du patient. Les vibrations appliquées au crâne sont transmises au saccule de l'oreille interne qui active le nerf cochléaire. Un tel système de traitement visant à stimuler indirectement le cortex auditif, via l'oreille interne, n'est pas adapté pour restaurer correctement l'audition pour les raisons déjà évoquées ci-dessus.

Dans le domaine de l'acuité visuelle, la cécité et les difficultés visuelles relèvent d'une problématique médicale majeure aujourd'hui dans la mesure où ces pathologies ont d'importantes répercussions sur la vie sociale des patients concernés qui représentent 0.5% de la population.

Il existe plusieurs causes possibles de cécité : malformative, infectieuse (toxoplasmose), traumatiques, tumorale (tumeur cérébrale), dégénérative (DMLA) vasculaire (diabete, stroke). Ces pathologies peuvent se trouver au niveau du globe oculaire, mais aussi sur toute la voie de transmission nerveuse (nerf optique, chiasma, bandelettes optiques, corps genouillés externes, radiations optiques, cortex visuel).

Dans les cas de difficultés visuelles sévères et de cécités non améliorables médicalement ou chirurgicalement, différentes solutions artificielles de restauration de la vision sont envisagées à ce jour :
S'il s'agit d'une pathologie du globe oculaire lui-même par atteinte de la cornée, ou du cristallin, la problématique n'est pas neurologique mais optique. Ainsi des lentilles artificielles sont implantées à la place de la cornée ou du cristallin.
S'il s'agit d'une pathologie des voies optiques neurologique, aucun système satisfaisant n'existe à ce jour. Cependant de multiples travaux de recherche sont menés. Soient ils concernent la restauration du circuit visuel par greffes soient ils concernent la mise au point de systèmes artificiels avec détection par caméra. L'image captée par la caméra est traitée pour aller ensuite activer les voies visuelles défectueuses naturelles via une interface électrique. Il s'agit donc de dispositifs de stimulation électrique du tissu neuronal.

La stimulation électrique peut être réalisée au niveau des cellules ganglionnaires de la rétine par la pose de plaques de 16 à 40 électrodes.

La stimulation électrique peut être réalisée directement au niveau du tissu neurologique (ganglion géniculés externes, cortex visuel cérébral) par la pose de plaques contenant jusqu'à 242 électrodes, soit posées sur la surface du cortex visuel, soit implantées au sein même du cortex.

Pour l'instant, les appareils les plus avancés n'ont fourni que des images rudimentaires en noir et blanc, encore insuffisantes pour se repérer dans un milieu inconnu. L'implantation de 242 électrodes par cerveau ne permet d'obtenir une vision que tunnel et pour un équivalent d'une matrice de rendu photo de 15 par 16 pixels. Plusieurs problèmes persistent expliquant cette faible efficacité :
Le cortex visuel primaire est principalement déployé sur la face médiale du cerveau, c'est-à-dire la face interne, où il est donc difficile d'aller positionner une plaque d'électrode profonde.
Les plaques avec électrodes qui pénètrent dans le cerveau offrent le meilleur rendement visuel actuel, mais elles sont hautement traumatiques pour le cortex, et la qualité de la connexion se détériore avec le temps à cause de l'apparition de couches isolantes et des mouvements pulsatiles physiologiques du cerveau à l'intérieur du crâne.
Les stimulations électriques sont toujours associées à des phénomènes de diffusion électrique dans le tissu notamment sensorineural. Ainsi, toute stimulation électrique du tissu neuronal adresse *in fine* plusieurs circuits neuronaux sans une spécificité suffisamment maîtrisée. Il est apparu que la densité d'électrode est aujourd'hui limitée non par limitation de fabrication, mais par les effets courants et court-circuits entre des électrodes trop proches.

Ainsi, les électrodes de stimulation électrique ne peuvent pas respecter fidèlement l'organisation anatomique et fonctionnelle des neurones (somatotopie) dans la mesure où les zones d'implantation sont très denses en neurones, ce qui là encore favorise l'activation de plusieurs circuits neuronaux simultanément. Ainsi, un tel système de traitement visant à stimuler indirectement le cortex visuel, n'est pas adapté pour restaurer correctement la vision pour les raisons déjà évoquées ci-dessus.

Dans un tout autre domaine, il a cependant récemment été démontré que l'utilisation d'ultrasons focalisés pulsés peut stimuler de façon non invasive les zones intactes motrices du cerveau. La neuromodulation ultrasonore se présente donc comme une technique favorable à la conception d'interfaces cerveau/machine non invasives de stimulation du cerveau.

A titre d'exemple, il a été montré chez le rat qu'une stimulation ultrasonore du cortex moteur engendrait une activité neuronale suffisante pour déclencher le comportement moteur du sujet (Tufail Y., Matyushov A., Baldwin N, Tauchmann ML, Georges J. Yoshihiro A. Tillery SI., Tyler WJ., Transcranial pulsed ultrasound stimulates intact brain circuits. Neuron, 2010 Jun 10 ;66(5) :681-94).

Un tel couplage de techniques ultrasonores avec la neurophysiologie a été évoqué pour la première fois en 2006 par Rvachev (Rvachev M.M. Alternative model of propagation of spikes along neurons, Physics 2006).

Basé sur ces premières études et dans le but de résoudre les problèmes reconnus des implants actuels, la présente invention a pour objet la fourniture d'un nouveau dispositif de traitement de la capacité sensorielle chez des patients.

L'invention vise particulièrement à procurer des moyens de traitement moins traumatisants et moins invasifs tout en étant plus précis et plus efficaces que les dispositifs actuellement connus de traitement de la capacité sensorielle d'un patient.

L'objet de l'invention vise donc à proposer un système de traitement de la capacité sensorielle d'un patient afin de remédier à une déficience, d'assister cette capacité sensorielle, voire d'augmenter cette capacité sensorielle.

L'invention vise notamment à proposer un système pour traiter au moins une capacité sensorielle d'une personne, à l'aide d'un dispositif de stimulation comprenant un convertisseur électronique d'un signal sensoriel en signaux électronique de commande d'au moins un transducteur pour émettre des signaux images du signal sensoriel. Selon l'invention, le dispositif de stimulation est un dispositif de stimulation ultrasonore directe du cortex sensoriel du cerveau du patient, et le dispositif de stimulation ultrasonore comporte :
- au moins un support implantable dans le crâne du patient et comprenant au moins une paroi interne,
- au moins un transducteur ultrasonore porté par le support et comportant des moyens pour émettre des ondes ultrasonores focalisées à travers la paroi interne du support, en direction d'une zone déterminée du cortex sensoriel du cerveau du patient en vue de générer une modulation de l'activité cérébrale dans ce cortex, ce transducteur ultrasonore étant piloté par le convertisseur électronique pour émettre des signaux ultrasonores focalisés, images du signal sensoriel.

Le système de traitement selon l'invention comporte également en combinaison l'une ou l'autre des caractéristiques additionnelles suivantes :
- un dispositif de stimulation ultrasonore comportant des moyens de focalisation et d'orientation variable des ondes ultrasonores émises en direction de la zone du cortex sensoriel visée,
- des moyens de focalisation et d'orientation des ondes ultrasonores comportant des moyens électroniques de balayage d'au moins une zone du cortex sensoriel à traiter par les ondes ultrasonores émises,
- la paroi inférieure du boîtier comprend une surface externe recouverte d'un matériau souple d'épaisseur variable pour procurer une interface de contact continu avec le cerveau ou la dure-mère et faciliter la propagation des ondes ultrasonores dans le cerveau,
- les moyens de réglage et de commande comportent une télécommande externe de contrôle et de pilotage sans fil du dispositif de stimulation ultrasonore,
- les moyens de réglage et de commande comportent un dispositif de commande externe raccordable au dispositif de stimulation ultrasonore,
- les moyens d'alimentation électrique comporte au moins une batterie d'accumulateur disposée dans le boîtier ou implantée sous-cutanée sur le corps du patient,
- un système de traitement comportant des électrodes au niveau d'une surface de contact externe de la paroi interne du boîtier pour contrôler l'activité électrique cérébrale et assurer la prévention de tout événement épileptique,
- un système de traitement comportant un interrupteur d'arrêt d'urgence dans la paroi supérieure du support,
- un système de traitement comportant un convertisseur électronique relié en entrée, à un système de fourniture d'une information sensorielle informatisée, provenant d'un appareil distant ou stockée dans ledit système.

Selon un premier objet, la présente invention propose un système de traitement de la surdité.

Le système de traitement de la surdité selon l'invention permet de réaliser une neuromodulation du cortex auditif primaire du cerveau d'un patient atteint de surdité par émission d'ondes ultrasonores de préférence focalisées sur une zone spécifique de faible surface ou volume dudit cortex auditif primaire, par exemple une surface ou un volume d'environ respectivement 1mm² ou 1mm³ du cortex auditif primaire en surface ou en profondeur, afin d'activer ce cortex de manière très sélective, au contact ou à distance du système, tout en permettant ainsi de respecter la somatotopie du cerveau mais aussi les aires superficielles du langage connexes au cortex auditif primaire.

Les ondes ultrasonores transmises au cerveau sont des ondes images des sons ambiants dans l'environnement du patient qui sont captés par un microphone qui peut être positionné vers l'extérieur du support de l'implant au travers de la paroi supérieure de celui-ci et de la peau du patient, ou encore déporté du support dans l'oreille externe du patient ou tout autre zone et reliée de façon filaire ou sans fil au dispositif de stimulation ultrasonore dans le support.

Bien entendu, la sensibilité de détection des sons ambiants et leur conversion sous forme d'ultrasons devront être adaptés pour chaque patient en fonction du niveau de surdité de ces patients.

Il est décrit par ailleurs un procédé non revendiqué de traitement de la surdité qui consiste essentiellement à stimuler, au moyen d'ondes ultrasonores focalisées émises dans le cerveau d'un patient, une zone déterminée du cortex auditif primaire du cerveau du patient.

Selon ce procédé non revendiqué, la fréquence des ondes ultrasonores émises est de préférence comprise dans la gamme de fréquence de 200 kHz à 10 MHz, et de préférence encore sur un mode pulsé.

De plus, de façon préférée, on focalise les ondes ultrasonores sur une surface ou sur un volume du cortex auditif primaire du cerveau du patient d'au moins respectivement 1mm² ou 1 mm³. Cette focalisation peut notamment être réalisée de façon dynamique par des moyens électroniques de balayage d'une ou plusieurs zones différentes du cortex auditif à traiter, de façon simultanée ou consécutive, par les ondes ultrasonores émises.

Par ailleurs, pour ajuster la puissance des ondes ultrasonores transmises et prévenir tout événement épileptique, on contrôle avantageusement en continu l'activité électrique cérébrale dans la zone d'émission ultrasonore du cortex visée.

Ce procédé non revendiqué peut tout particulièrement être mis en oeuvre à l'aide d'un système de traitement de la surdité tel que décrit précédemment après implantation du support dans un trou de trépan préalablement formé dans la boîte crânienne du patient au droit de la zone du cortex auditif primaire du cerveau à traiter.

Selon un autre objet, la présente invention propose un système de traitement de l'acuité visuelle d'un patient.

Selon cet objet, le système de traitement comporte en combinaison, l'une et/ou des caractéristiques additionnelles suivantes :
- le capteur est un système d'acquisition d'images et le transducteur ultrasonore focalise les ondes ultrasonores en direction d'une zone déterminée du cortex cérébral visuel du patient,
- le dispositif de stimulation ultrasonore comporte deux boîtiers implantables dans lesquels sont montés les transducteurs ultrasonores, chaque boitier implantable présentant une face interne à travers laquelle sont émises les ondes ultrasonores présentant une face interne prolongeant la face interne du boitier et à travers laquelle sont émises également les ondes ultrasonores focalisées.

Diverses autres caractéristiques ressortent de la description faite ci-dessous en référence aux dessins annexés qui montrent, à titre d'exemples non limitatifs, des formes de réalisation de l'objet de l'invention.

Sur les figures annexées :
- La **figure 1** représente une coupe partielle du cerveau sur une image par résonance magnétique montrant la zone cérébrale du Gyrus de Heschel formant le cortex auditif primaire et le principe d'activation de cette zone cérébrale à l'aide d'un système selon l'invention
- La **figure 2** représente schématiquement en coupe le système de traitement de la surdité de l'invention implanté dans le crâne d'un patient pour stimuler le cortex auditif primaire du cerveau du patient
- La **figure 3** représente schématiquement en coupe longitudinale un système de traitement de la surdité conforme à l'invention dans un premier mode de réalisation
- La **figure 4** représente schématiquement en coupe longitudinale un système de traitement de la surdité conforme à l'invention dans un second mode de réalisation
- Les **figures 5A** et **5B** représentent respectivement deux variantes de réalisation de la surface inférieure du boîtier implantable du système de traitement de l'invention,
- Les **figures 6A** et **6B** représentent respectivement deux variantes de réalisation de la surface supérieure du boîtier implantable du système de traitement de l'invention.
- La **figure 7** est une vue schématique en coupe d'un système de traitement de la vision conforme à l'invention, implantée dans le crâne d'un patient pour stimuler le cortex cérébral visuel d'un patient.
- La **figure 8** est une vue schématique postérieure du crâne d'un patient équipé d'un système de traitement de la vision conforme à l'invention.
- Les **figures 9** et **10** sont des vues respectivement externe et interne d'un exemple de réalisation d'un système de traitement de la vision conforme à l'invention.

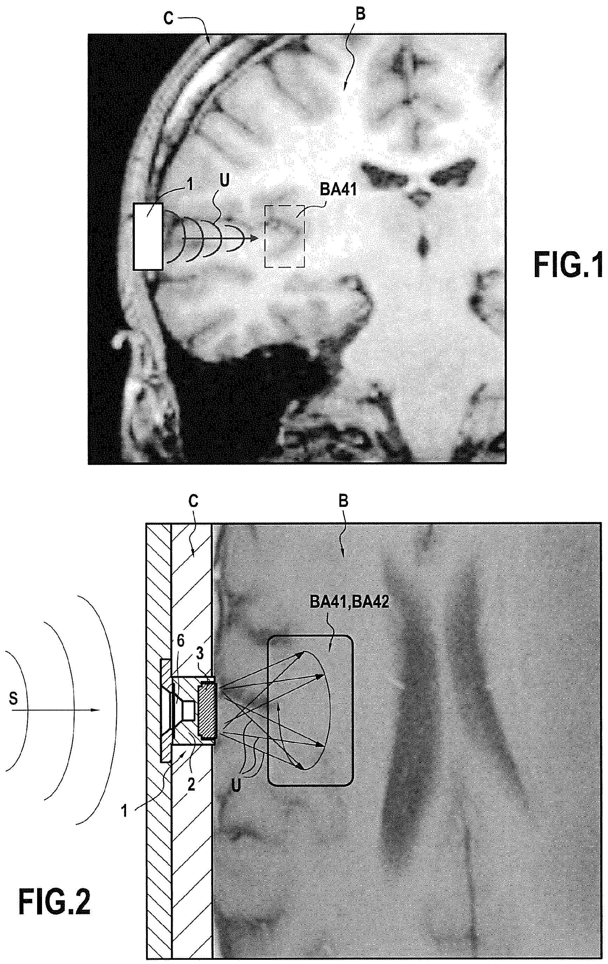

La présente invention propose une nouvelle approche de traitement des capacités sensorielles des patients. Les **figures 1** à **6B** décrivent un exemple d'application de l'objet de l'invention au traitement de l'audition en tant que capacité sensorielle et en particulier de la surdité chez des patients atteints de surdité sensorineurale et/ou transmissive. Cette nouvelle approche consiste, comme schématisé sur les **figures 1** et **2****,** en une stimulation ultrasonore du cortex auditif primaire du cerveau **B** d'un patient par l'intermédiaire d'un système de traitement **1** implantable dans le crâne **C** du patient en sous cutanée et comprenant un dispositif de stimulation ultrasonore **3** apte à émettre des ondes ultrasonores **U** focalisées sur la zone du Gyrus de Heschel **(BA41)** et/ou du planum temporal **(BA42)** qui forment le cortex auditif primaire du cerveau **B** humain. Les ondes ultrasonores **U** émises sont des ondes pulsées, modulées par un convertisseur électronique du dispositif de stimulation ultrasonore **3** en fonction d'un signal sensoriel et plus précisément des sons **S** extérieurs par exemple, perçus par au moins un capteur tel qu'un microphone **6** relié au dit dispositif de stimulation ultrasonore **3.**

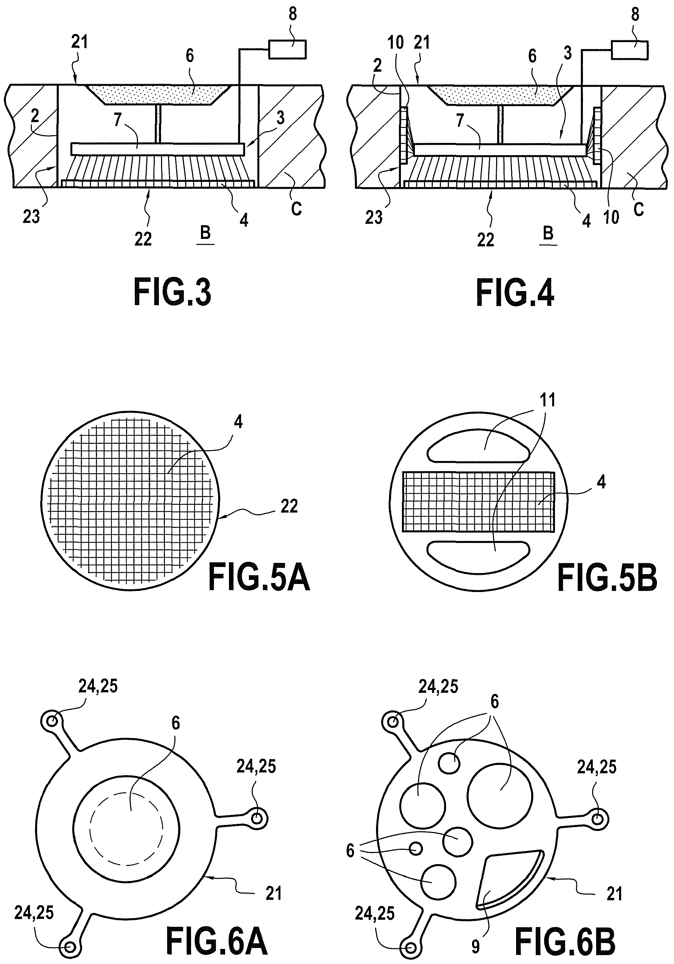

Le système de traitement de la surdité **1** de l'invention comporte, comme représenté sur les **figures 2** à **4****,** un support tel qu'un boîtier **2** d'implantation dans le crâne **C** du patient, notamment dans un trou de trépan pratiqué au droit de la zone du cerveau **B** formant le cortex auditif primaire. Ce boîtier **2** peut être de forme quelconque, notamment de section circulaire ou rectangulaire. Avantageusement, le boîtier **2** est réalisé en un matériau non ferromagnétique pour assurer une compatibilité IRM (Imagerie par Résonance Magnétique). Il comprend une paroi supérieure ou externe **21** et une paroi inférieure ou interne **22** toutes deux reliées par une paroi périphérique **23** et des moyens de fixation **24** du boîtier **2** sur le crâne du patient. Ces moyens de fixations **24** peuvent consister en des pattes de fixation **25** s'étendant sur les côtés de la paroi supérieure **21** du boîtier **2** et dans lesquelles sont insérées des vis tel que représenté sur les **figures 6A** et **6B****,** ou encore en des stries de vissage sur la surface externe de la paroi périphérique **23** pour permettre un vissage du boîtier **2** dans le trou de trépan du crâne. En position fixée, le boîtier **2** traverse complètement le crâne **C** pour déboucher directement par sa paroi inférieure **22** sur le cerveau **B,** comme illustré aux **Fig. 3** et **4****.**

A l'intérieur du boîtier **2** est placé un dispositif **3** de stimulation ultrasonore. Ce dispositif de stimulation ultrasonore 3 comporte au moins un, et de préférence une pluralité de transducteurs ultrasonores **4** montés et orientés dans le boîtier **2** pour émettre des ondes ultrasonores **5** pulsées au travers de la paroi inférieure **22** du boîtier **2** pour générer une dépolarisation neuronale et donc une activité cérébrale dans le cortex auditif primaire en fonction des sons ambiants **S** perçus par le ou les microphones **6.** Comme expliqué ci-dessus, le dispositif de stimulation ultrasonore **3** comporte des moyens pour émettre des ondes ultrasonores focalisées **U** à travers la paroi inférieure **22** du boitier **2,** sans traverser le crâne **C** en direction d'une zone déterminée du cortex auditif primaire du cerveau du patient. La paroi inférieure **22** est donc réalisée en un matériau perméable aux ondes ultrasonores.

De préférence, comme représenté sur la **figure 5A****,** la paroi inférieure **22** du boîtier **2** est formée intégralement ou à tout le moins partiellement par des micro-transducteurs ultrasonores **4** couvrant au moins 50% de la surface de ladite paroi inférieure **22** du boîtier **2.**

La paroi inférieure **22** du boîtier **2** peut dans un mode de réalisation de l'invention comprendre une surface externe recouverte d'un matériau souple d'épaisseur variable afin de procurer une interface de contact continu avec le cerveau ou la dure-mère dans le crâne **C** patient et ainsi faciliter la propagation des ondes ultrasonores **U** dans le cerveau **B.** La paroi inférieure **22** du boîtier **2** est réalisée en un matériau biocompatible compte tenu de son implantation à l'intérieur du crâne **C.**

Selon l'invention, les transducteurs ultrasonores **4** peuvent être choisis parmi des éléments piézo-composites, piézo-céramiques, des éléments ultrasonores capacitifs (CMUT), des éléments de polyfluorure de vinylidène (PVDF), ou autres aptes à émettre des ondes ultrasonores pulsées.

Les ondes ultrasonores **U** émises par les transducteurs disposés dans le boîtier sont des ondes ultrasonores pulsées qui sont focalisées en direction du cortex auditif primaire (zones **BA41, BA42)** du cerveau pour moduler spécifiquement et sélectivement sur une zone de très faible surface l'activité du cerveau. Les ondes ultrasonores focalisées **U** atteignent ainsi directement cette zone du cerveau **B** sans traverser le crâne **C,** dans la mesure où la paroi inférieure **22** du boîtier **2** qui est traversée par les ondes ultrasonores est en contact avec le cerveau **B.** La fréquence d'émission des ondes ultrasonores par le dispositif de l'invention est de préférence de l'ordre de 200 kHz à 10 MHz.

Cette gamme fréquentielle est particulièrement adaptée et nécessaire pour adapter les paramètres d'émission et de traitement du système **1** de l'invention à tout patient et à toutes configurations anatomiques et notamment pour permettre une orientation correcte du faisceau d'ultrasons et de la focalisation de ce faisceau lors du traitement en faisant varier la différence de phase entre les émetteurs, en rajoutant des filtres, des lentilles acoustiques ou une combinaison de ces moyens.

Dans l'exemple illustré sur les dessins, le dispositif de stimulation ultrasonore **3** est placé à l'intérieur d'un support se présentant sous la forme d'un boitier **2** équipé de moyens de fixation **25** sur le crâne du patient. Il est à noter qu'il peut être envisagé de placer le dispositif de stimulation ultrasonore **3** à l'intérieur d'un support réalisé sous la forme d'une membrane insérée à l'intérieur d'un crâne **C** et comportant une face interne à travers laquelle les ondes ultrasonores sont émises et en contact avec le cerveau ou la dure-mère.

Le dispositif de stimulation ultrasonore **3** comporte également au moins un microphone **6** adapté pour capter les sons ambiants audibles dans l'environnement direct du patient implanté. Ce microphone **6** peut être selon une première forme préférée de réalisation positionné dans la paroi supérieure **21** du boîtier **2** comme représenté sur la **figure 6A****.** Le cas échéant, comme représenté sur la **figure 6B****,** une multiplicité de microphones **6** peut être employée pour capter avec chaque microphone **6** une plage de longueurs d'ondes sonores déterminée.

Il est également envisageable en cas de microphone unique **6** de démembrer le microphone **6** du boîtier **2,** notamment pour le positionner dans le pavillon auriculaire du patient et transmettre les informations de manière filaire ou sans fil au boîtier **2** implanté dans le crâne C du patient. De la même façon, on peut également utiliser deux microphones **6** disposés chacun dans un pavillon auriculaire du patient pour réaliser un enregistrement stéréo des sons **S** ambiants.

Le ou les microphone(s) **6** sont reliées sur l'intérieur du boîtier **2** à un convertisseur électronique **7** de décodage des sons perçus **S** en signaux électroniques de commande des transducteurs ultrasonores **4.** Ainsi, les ondes ultrasonores **U** émises par les transducteurs ultrasonores **4** forment par le biais du convertisseur, des signaux ultrasonores images des sons **S** captés par le ou les microphone(s) **6.**

Il ressort de la description qui précède que le convertisseur électronique **7** est relié en entrée au microphone **6** pour assurer la conversion des sons captés par le microphone **6,** en signaux de commande des transducteurs ultrasonores **4.** Bien entendu, le convertisseur électronique **7** peut assurer la conversion de sons, non pas captés par un microphone, mais générés par un système de simulation intégré ou déporté par rapport au dispositif de stimulation **3.** Selon cet exemple de réalisation, le convertisseur électronique **7** est relié en entrée, au système de simulation, pour convertir les signaux sonores simulés, en signaux de commande des transducteurs ultrasonores **4.**

Dans un mode de réalisation, l'information auditive peut provenir directement de données informatisée en provenance de fichiers informatiques, sans nécessité de système de détection auditive (microphone). Ceci permet d'apporter, de façon réelle au cerveau, une information sensorielle informatisée totalement virtuelle. Les dits fichiers informatiques peuvent être stockés dans un téléphone portable ou être reçue directement par internet en temps réel dans ce téléphone portable. Ainsi le convertisseur électronique **7** est relié en entrée, à un système de fourniture d'une information sensorielle informatisée, provenant d'un appareil distant ou stockée dans ledit système.

Ce convertisseur électronique **7** peut être avantageusement réalisé sous la forme d'un circuit intégré et/ou d'une carte électronique comportant au moins un microprocesseur et les composants de conversion de signaux du ou des microphones **6** en signaux de pilotage des transducteurs ultrasonores **4.** Dans ce cas, la carte électronique forme une intelligence de contrôle du système de traitement et peut également supporter toute autre fonction de réglage et de commande du système de traitement **1** et de ces composants.

En particulier, le système de traitement **1** de l'invention comporte également des moyens de commande du dispositif de stimulation ultrasonore **3.** Ces moyens de commande et réglage peuvent être réalisés sur la carte électronique précitée et comprendre des moyens de communication filaire ou sans fil avec un dispositif de contrôle externe tel qu'une télécommande interagissant avec le dispositif de stimulation ultrasonore pour permettre la mise en fonction et l'arrêt des microphone(s) **6,** du convertisseur et des transducteurs **4.** Les moyens de réglage et de commande peuvent également comporter un dispositif de commande externe, par exemple un moniteur, raccordable au dispositif de stimulation ultrasonore **3** au travers de la peau **P** du crâne **C** par un connecteur transdermique placé dans la paroi supérieure **21** du boîtier **2.**

Il peut également être envisagé un contrôle et réglage du système de traitement **1** par le biais d'un téléphone portable et d'une application informatique enregistrée sur celui-ci une mémoire ROM de celui-ci et permettant réglage et contrôle du système de traitement sans fil, par exemple selon les protocoles de communication définis par le groupe IEEE 802.11 (ISO/CEI 8802-11), plus connu sous la contraction « Wifi », ou encore dans le groupe IEEE 802.15, également plus connu sous le nom anglais « Bluetooth ».

Le système de traitement **1** de surdité de l'invention comporte enfin des moyens **8** d'alimentation électrique du dispositif de stimulation ultrasonore, des microphones, des transducteurs ultrasonores **4** et des moyens de commande de ce dispositif. De façon classique, ces moyens d'alimentation électrique **8** peuvent consister en des batteries d'accumulateur disposées dans le boîtier **2** ou encore par une batterie sous-cutanée implantée sous la peau du crâne ou encore à distance de la tête du patient, sous la peau de celui-ci par exemple dans la région thoracique, comme cela est habituellement réalisé en matière de chirurgie du coeur pour l'implantation de pacemakers. Le cas échéant, une telle batterie peut alors être chargée par un système de chargement externe tel qu'un système magnétique.

Il est également possible de prévoir, comme représenté sur la **figure 6B****,** un interrupteur d'arrêt d'urgence **9** directement dans la paroi supérieure **21** du boîtier **2** au niveau de la surface du crâne du patient.

De préférence, le système de traitement **1** de l'invention est compatible avec des analyses par imagerie de résonance magnétique. C'est la raison pour laquelle le boîtier **2,** mais aussi tous les transducteurs ultrasonores **4,** le ou les microphone(s) **6,** le convertisseur et autres moyens de commande et d'alimentation sont de préférence constitué de matériaux non ferromagnétiques.

Selon un mode de réalisation préféré du système de traitement **1** de l'invention, celui-ci comporte des moyens de focalisation et d'orientation des ondes ultrasonores **U** émises en direction de la zone du cortex auditif primaire visée. Ils peuvent, par exemple, résulter d'une focalisation dynamique (beam steering) en jouant sur la différence de phase entre émetteurs, en rajoutant des filtres, des capaciteurs, des lentilles ou une combinaison de ces moyens.

Ils peuvent par exemple résulter d'une géométrie particulière de la face d'émission des transducteurs ultrasonores **4,** notamment d'une concavité particulière de cette face d'émission ou de la paroi inférieure **22** du boîtier **2** portant ces transducteurs ultrasonores **4.**

Ces moyens de focalisation et d'orientation variables des ondes ultrasonores **U** peuvent également comporter des moyens électroniques de balayage d'une ou plusieurs zones du cortex auditif du cerveau **B** du patient par les ondes ultrasonores **U** émises par les transducteurs ultrasonores.

Dans un autre mode de réalisation du système de traitement **1** de l'invention représenté à la figure **4****,** le système de traitement **1** peut comprendre au moins un émetteur ultrasonore additionnel **10** placé dans l'épaisseur du boîtier **2** pour induire et transmettre une vibration osseuse du crâne. Un tel émetteur ultrasonore additionnel **10,** plaqué par exemple comme représenté à la figure **4** sur la paroi périphérique **23** du boîtier **2** peut s'avérer particulièrement utile dans des cas de surdité transmissive pure ou mêlée à une surdité sensorineurale. Dans ce mode de réalisation, l'élément ultrasonore additionnel **10** est lui aussi piloté alors par le convertisseur électronique dans le boîtier **2.**

Dans un autre mode de réalisation du représenté à la **figure 5B****,** le système de traitement **1** peut comprendre en outre des électrodes de contact **11** dans la paroi inférieure **22** du boîtier **2,** par exemple de part et d'autre des transducteurs ultrasonore **4** ou au sein même des émetteurs ultrasonores pour contrôler l'activité cérébrale électrique et assurer la prévention de tout événement épileptique.

Dans ce cas, le contrôle du signal électrique cortical est effectué par l'intermédiaire des électrodes **11** connectée à une carte électronique de contrôle placée dans le boîtier **2,** et de préférence intégrée ou associée au convertisseur pour former l'intelligence de pilotage du système de traitement **1.** En cas de signal anormal épileptogénique, les paramètres d'émission ultrasonore peuvent être automatiquement stoppés pour des raisons de sécurité ou modifiés pour émettre un signal ultrasonore inhibiteur.

Le système **1** de traitement de la surdité de l'invention permet ainsi la stimulation directe et la neuromodulation de l'activité cérébrale du cortex auditif primaire du cerveau **B** d'un patient atteint de surdité par émission d'ondes ultrasonores **U** focalisées et pulsées sur une surface très localisée de 1 mm² environ, du cortex auditif primaire du cerveau **B** du patient. Une telle modulation du cortex auditif primaire résulte de la transmission d'ondes ultrasonores **U** images des sons ambiants **S** perçus par les microphones **6** du système de traitement **1** et permettent ainsi de traiter de façon sensible les pathologies de surdité sensorineurale chez les patients qui en sont atteints.

Le système de traitement **1** de l'invention permet également de contrôler l'activité cérébrale du cerveau **B** du patient traité en cours de traitement par le biais d'électrodes de contact **11** et ainsi d'ajuster la puissance des ondes ultrasonores **U** transmises et prévenir tout événement épileptique. On contrôle ainsi avantageusement en continu l'activité électrique cérébrale dans la zone d'émission ultrasonore du cortex visé.

Le système de traitement **1** de l'invention permet également de contrôler la diffusion des ondes ultrasonores dans le cerveau **B** du patient traité avant et pendant le traitement par l'enregistrement des ondes ultrasonores réfléchies renvoyées par le cerveau, afin de connaître l'index d'atténuation acoustique du cerveau du patient, de détecter d'éventuelles modifications structurales du tissu, de détecter d'éventuelles modifications de la perfusion cérébrale pour moduler et régler au mieux l'intensité des ondes émises.

Le système de traitement de la surdité de l'invention procure une solution nouvelle de traitement de la surdité respectant la somatotopie du cerveau des patients sans présenter les inconvénients des implants de traitement connus à ce jour et procurant une plus grande flexibilité et précision de traitement sans toucher à l'appareil auditif naturel défectueux du patient.

Il convient par ailleurs de noter qu'un système de traitement analogue à celui de l'invention peut être envisagé pour stimuler le cortex cérébral visuel. Dans ce cas, la structure du système de traitement diffère alors essentiellement de celle du système de l'invention en ce que les microphones sont remplacés par au moins un dispositif d'acquisition d'images, par exemple une micro-caméra, relié au dispositif de commande dont le convertisseur interne convertie le signal image provenant de ce dispositif d'acquisition en signal de modulation et de commande des émetteurs ultrasonores qui focalisent leurs ondes en direction du cortex cérébral visuel.

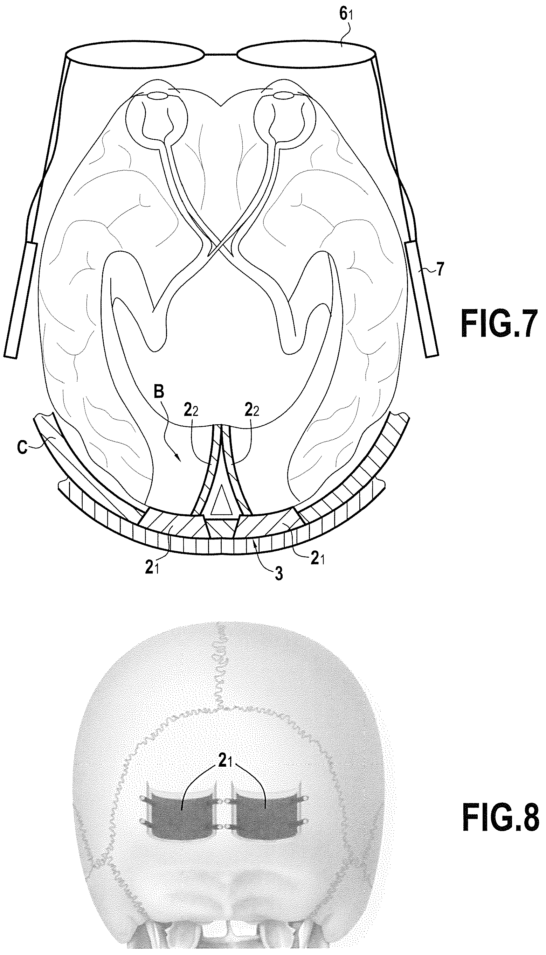

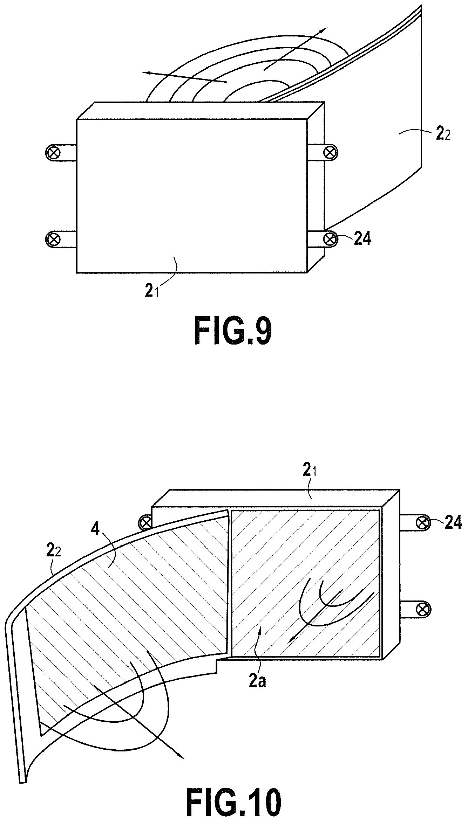

Les figures **8** à **10** illustrent cette autre application préférée de l'objet à l'invention au traitement de la vue en tant que capacité sensorielle d'un patient. Selon cette application, le dispositif de stimulation ultrasonore **3** comporte toutes les caractéristiques techniques décrites en relation de l'application au traitement de la surdité mais appliquées au traitement de la vision.

Selon cette application, le dispositif de stimulation ultrasonore **3** comporte deux boitiers implantables **2₁,** dans lesquels sont montés les transducteurs ultrasonores **4.** Chaque boitier implantable **2₁** est pourvu d'une aile semi-rigide **2₂** de forme concave adaptée pour la stimulation du cortex cérébral visuel médian comme cela apparaît à la figure **7****.** Le boitier **2₁** et l'aile semi-rigide **2₂** présentent une face interne **2a** à travers de laquelle sont émises les ondes ultrasonores focalisées, générées par des transducteurs ultrasonores **4** montées dans le boitier **2₁** et l'aile semi-rigide **2₂.** Un tel boitier 6₁ est implanté dans un trou de craniectomie préalablement formé dans la boite crânienne du patient au droit de la zone du cortex visuel primaire du cerveau à traiter. L'aile semi-rigide **2₂** est glissée en inter hémisphérique après ouverture de la dure-mère. Le boitier **2₁** est équipé de moyens de fixation **24** sur le crâne du patient.

Comme expliqué en relation des figures **1** à **6B****,** les transducteurs ultrasonores **4** sont répartis sur la face interne **2a** des boitiers implantables pour émettre des ondes ultrasonores focalisées dont la fréquence est comprise entre 200 KHz et 10 MHz. Les ondes ultrasonores sont focalisées sur une zone spécifique de faible surface du cortex visuel primaire, par exemple une surface d'environ 1 mm² du cortex visuel primaire, afin d'activer ce cortex visuel de manière très sélective tout en permettant de respecter la somatotopie du cerveau. Comme expliqué ci-dessus, la focalisation des ondes ultrasonores peut être réalisée de façon dynamique par des moyens électroniques de balayage d'une ou de plusieurs zones différentes du cortex visuel à traiter, de façon simultanée ou consécutive, par les ondes ultrasonores émises.

Par ailleurs, pour ajuster la puissance des ondes ultrasonores transmises et prévenir tout événement épileptique, il est prévu de contrôler en continu l'activité cérébrale dans la zone d'émission ultrasonore du cortex visuel visée.

Les transducteurs ultrasonores **4** sont pilotés comme expliqué précédemment, par le convertisseur électronique **7,** de sorte que les signaux ultrasonores focalisées **U** soient une image d'un signal sensoriel, à savoir un signal visuel dans l'application visée. Un tel signal visuel est soit capté par un capteur **6₁,** tel qu'un système d'acquisition d'images soit généré par un système de fourniture d'une information sensorielle informatisée, totalement virtuelle. Dans l'exemple illustré à la figure **7****,** le système d'acquisition d'images **6₁** comporte au moins une caméra pour prendre des images dans l'environnement du patient. La caméra est reliée en entrée au convertisseur électronique **7** adapté pour décoder et traiter l'information visuelle perçue par la caméra et la transformer en signaux de commande pour les transducteurs ultrasonores **4.**

Dans un mode de réalisation, l'information visuelle peut provenir directement de données informatisée en provenance de fichiers informatiques, sans nécessité de système de détection visuelle (caméra). Ceci permet d'apporter, de façon réelle au cerveau, une information sensorielle informatisée totalement virtuelle. Les dits fichiers informatiques peuvent être stockés dans un téléphone portable ou être reçue directement par internet en temps réel dans ce téléphone portable. Ainsi le convertisseur électronique **7** est relié en entrée, à un système de fourniture d'une information sensorielle informatisée, provenant d'un appareil distant ou stockée dans ledit système.

Selon une variante de réalisation, le convertisseur électronique **7** comprend des moyens de communication filaire ou sans fil, avec le dispositif de stimulation ultrasonore **3** et en particulier, avec les deux boitiers implantables **2₁.** Par exemple, la caméra **6₁** peut être intégrée à des lunettes **L** qui portent également le convertisseur électronique **7.**

Le système de traitement **1** selon l'invention, comporte également des moyens de commande du dispositif de stimulation ultrasonore **3** permettant la mise en fonction et l'arrêt du dispositif de stimulation ultrasonore **3.** Comme indiqué précédemment, ces moyens de commande peuvent être réalisés de différentes manières, comme par exemple, à l'aide d'un système de commande externe raccordé au dispositif de stimulation ultrasonore **3,** à travers la peau du crâne **C** ou à l'aide d'une transmission sans fil.

Le système de traitement **1** de la vision comporte, bien entendu, des moyens **8** d'alimentation électrique du dispositif de stimulation ultrasonore **3,** de la caméra, et du convertisseur électronique **7.** De façon classique, ces moyens d'alimentation électrique **8** peuvent consister en des accumulateurs d'énergie disposés dans les boitiers **2** ou encore par une batterie sous-cutanée implantée sous la peau du crâne ou à distance de la tête du patient. Une telle batterie peut aussi être chargée par un système de chargement externe tel qu'un système magnétique.

Le système de traitement **1** de l'invention permet également de contrôler l'activité cérébrale du cerveau **B** du patient traité en cours de traitement par le biais d'électrodes de contact **11** et ainsi d'ajuster la puissance des ondes ultrasonores **U** transmises et prévenir tout événement épileptique. On contrôle ainsi avantageusement en continu l'activité électrique cérébrale dans la zone d'émission ultrasonore du cortex visé.

Le système de traitement **1** de l'invention permet également de contrôler la diffusion des ondes ultrasonores dans le cerveau **B** du patient traité avant et pendant le traitement par l'enregistrement des ondes ultrasonores réfléchies renvoyées par le cerveau, afin de connaître l'index d'atténuation acoustique du cerveau du patient, de détecter d'éventuelles modifications structurales du tissu, de détecter d'éventuelles modifications de la perfusion cérébrale pour moduler et régler au mieux l'intensité des ondes émises.

## Revendications

1. Système **(1)** pour traiter au moins une capacité sensorielle d'une personne, comprenant un dispositif de stimulation comprenant un convertisseur électronique (**7**) d'un signal sensoriel en signaux électroniques de commande d'au moins un transducteur pour émettre des signaux images du signal sensoriel, le dispositif de stimulation étant un dispositif de stimulation ultrasonore (**3**) directe du cortex sensoriel du cerveau (**B**) du patient, le dispositif de stimulation ultrasonore comportant :
- au moins un support (**2,2₁**) implantable dans le crâne (**C**) du patient et comprenant au moins une paroi interne (**22**) ;
**caractérisé en ce que** le dispositif de stimulation ultrasonore comprend
- au moins un transducteur ultrasonore (**4**) porté par le support (**2**) et comportant des moyens pour émettre des ondes ultrasonores focalisées (**U**) à travers la paroi interne (**22**) du support, sans traverser le crâne (**C**) en direction d'une zone déterminée du cortex sensoriel du cerveau (**B**) du patient en vue de générer une modulation de l'activité cérébrale dans ce cortex, ce transducteur ultrasonore (**4**) étant piloté par le convertisseur électronique pour émettre des signaux ultrasonores focalisés (**U**), images du signal sensoriel.

2. Système de traitement selon la revendication 1 **caractérisé en ce que** le convertisseur électronique (**7**) est relié en entrée à au moins un capteur (**6, 6₁**) adapté pour capter un signal sensoriel (**5**) présent dans l'environnement de la personne.

3. Système de traitement selon la revendication 2 **caractérisé en ce que** le capteur (**6**) est un microphone adapté pour capter les sons ambiants et **en ce que** le transducteur ultrasonore (**4**) focalise les ondes ultrasonores en direction d'une zone déterminée du cortex auditif primaire du cerveau (**B**) du patient.

4. Système de traitement selon l'une des revendications 1 à 3 **caractérisé en ce que** le support comporte un boitier (**2**) équipé de moyens de fixation (**24**) sur le crâne du patient et de préférence dans un trou de trépan, le boitier (**2**) comportant une paroi externe (**21**) reliée à la paroi interne (**22**) par l'intermédiaire d'une paroi périphérique (**23**).

5. Système de traitement selon la revendication 2 **caractérisé en ce que** le capteur (**6₁**) est un système d'acquisition d'images et **en ce que** le transducteur ultrasonore (**4**) focalise les ondes ultrasonores en direction d'une zone déterminée du cortex cérébral visuel du patient.

6. Système de traitement selon la revendication 1 ou 5 **caractérisé en ce que** le dispositif de stimulation ultrasonore (**3**) comporte deux boitiers implantables (**2₁**) dans lesquels sont montés les transducteurs ultrasonores (**4**), chaque boitier implantable (**2₁**) présentant une face interne à travers laquelle sont émises les ondes ultrasonores focalisées, chaque boitier implantable étant pourvu d'une aile semi-rigide (**2₂**) concave présentant une face interne prolongeant la face interne du boitier et à travers laquelle sont émises également les ondes ultrasonores focalisées.

7. Système (**1**) de traitement selon l'une des revendications 1 à 6, **caractérisé en ce que** le dispositif de stimulation ultrasonore (**3**) comporte des moyens de focalisation et d'orientation variable des ondes ultrasonores (**U**) émises en direction de la zone du cortex sensoriel visée.

8. Système (**1**) de traitement selon la revendication 7, **caractérisé en ce que** les moyens de focalisation et d'orientation des ondes ultrasonores (**3**) comportent des moyens électroniques de balayage d'au moins une zone du cortex sensoriel à traiter par les ondes ultrasonores (**U**) émises.

9. Système (**1**) de traitement selon l'une des revendications 1 à 8, **caractérisé en ce que** la paroi inférieure (**22**) du support (**2**) comprend une surface externe recouverte d'un matériau souple d'épaisseur variable pour procurer une interface de contact continu avec le cerveau (**B**) ou la dure-mère et faciliter la propagation des ondes ultrasonores (**U**) dans le cerveau (**B**).

10. Système (**1**) de traitement selon l'une des revendications 1 à 9, **caractérisé en ce que** les moyens de réglage et de commande comportent une télécommande externe de contrôle et de pilotage sans fil du dispositif de stimulation ultrasonore.

11. Système (**1**) de traitement selon l'une des revendications 1 à 10, **caractérisé en ce que** les moyens de réglage et de commande comportent un dispositif de commande externe raccordable au dispositif de stimulation ultrasonore (3).

12. Système (**1**) de traitement selon l'une des revendications 1 à 11, **caractérisé en ce que** les moyens d'alimentation électrique (**8**) comporte au moins une batterie d'accumulateur disposée dans le boîtier (**2**) ou implantée sous-cutanée sur le corps du patient.

13. Système (**1**) de traitement selon l'une des revendications précédentes, **caractérisé en ce qu'**il comporte des électrodes (**11**) au niveau d'une surface de contact externe de la paroi interne (**22**) du boîtier(**2**) pour contrôler l'activité électrique cérébrale et assurer la prévention de tout événement épileptique.

14. Système (**1**) de traitement selon l'une des revendications précédentes, **caractérisé en ce qu'**il comporte un interrupteur d'arrêt d'urgence (9) dans la paroi supérieure du support.

15. Système (**1**) de traitement selon la revendication 1 **caractérisé en ce que** le convertisseur électronique (**7**) est relié en entrée, à un système de fourniture d'une information sensorielle informatisée, stockée dans ledit système, ou provenant d'un appareil distant.
Système (**1**) pour traiter au moins une capacité sensorielle d'une personne, à l'aide d'un dispositif de stimulation comprenant :
• un convertisseur électronique (**7**) d'un signal sensoriel en signaux électronique de commande d'au moins un transducteur pour émettre des signaux images du signal sensoriel,
• un dispositif de stimulation ultrasonore (**3**) directe du cortex sensoriel du cerveau (**B**) du patient, comportant :
- au moins un support (**2**) implantable dans le crâne (**C**) du patient et comprenant au moins une paroi interne (**22**),
- au moins un transducteur ultrasonore (**4**) porté par le support (**2**) et comportant des moyens pour émettre des ondes ultrasonores focalisées (U) à travers la paroi interne (**22**) du support, en direction d'une zone déterminée du cortex sensoriel du cerveau (**B**) du patient en vue de générer une modulation de l'activité cérébrale dans ce cortex, ce transducteur ultrasonore (**4**) étant piloté par le convertisseur électronique pour émettre des signaux ultrasonores focalisés (**U**), images du signal sensoriel.

## Patentansprüche

1. System (1) zur Behandlung mindestens einer sensorischen Fähigkeit einer Person, umfassend eine Stimulationsvorrichtung, umfassend einen elektronischen Wandler (7) eines sensoriellen Signals in elektronische Signale zur Steuerung mindestens eines Wandlers, um Bildsignale des sensoriellen Signals zu entsenden, wobei die Stimulationsvorrichtung eine Vorrichtung zur direkten Ultraschallstimulation (3) des sensoriellen Kortex des Gehirns (B) des Patienten ist, wobei die Ultraschallstimulationsvorrichtung umfasst:
- mindestens einen Träger (2, 2₁), der in den Schädel (C) des Patienten implantierbar ist, umfassend mindestens eine Innenwand (22),
**dadurch gekennzeichnet, dass** die Ultraschallstimulationsvorrichtung umfasst:
- mindestens einen Ultraschallwandler (4), der von dem Träger (2) getragen wird, und Mittel umfasst, um fokussierte Ultraschallwellen (U) durch die Innenwand (22) des Trägers zu entsenden, ohne den Schädel (C) in Richtung einer bestimmten Zone des sensoriellen Kortex des Gehirns (B) des Patienten zu durchqueren, um eine Modulation der Gehirntätigkeit in diesem Kortex zu erzeugen, wobei dieser Ultraschallwandler (4) von dem elektronischen Wandler gesteuert wird, um fokussierte Ultraschallsignale (U), Bilder des sensoriellen Signals, zu entsenden.

2. Behandlungssystem nach Anspruch 1, **dadurch gekennzeichnet, dass** der elektronische Wandler (7) am Eingang mit mindestens einem Sensor (6, 6₁) verbunden ist, der dazu geeignet ist, ein sensorielles Signal (5), das in der Umgebung der Person vorhanden ist, zu erfassen.

3. Behandlungssystem nach Anspruch 2, **dadurch gekennzeichnet, dass** der Sensor (6) ein Mikrofon ist, das dazu vorgesehen ist, die Raumtöne zu erfassen, und dass der Ultraschallwandler (4) die Ultraschallwellen in Richtung einer bestimmten Zone des primären auditiven Kortex des Gehirns (B) des Patienten fokussiert.

4. Behandlungssystem nach einem der Ansprüche 1 bis 3, **dadurch gekennzeichnet, dass** der Träger ein Gehäuse (2) umfasst, das mit Befestigungsmitteln (24) auf dem Schädel des Patienten und vorzugsweise in einem Bohrloch versehen ist, wobei das Gehäuse (2) eine Außenwand (21) umfasst, die mit der Innenwand (22) mit Hilfe einer Umfangswand (23) verbunden ist.

5. Behandlungssystem nach Anspruch 2, **dadurch gekennzeichnet, dass** der Sensor (6₁) ein Bilderaufnahmesystem ist, und dass der Ultraschallwandler (4) die Ultraschallwellen in Richtung einer bestimmten Zone des visuellen zerebralen Kortex des Patienten fokussiert.

6. Behandlungssystem nach Anspruch 1 oder 5, **dadurch gekennzeichnet, dass** die Ultraschallstimulationsvorrichtung (3) zwei implantierbare Gehäuse (2₁) umfasst, in denen die Ultraschallwandler (4) montiert sind, wobei jedes implantierbare Gehäuse (2₁) eine Innenseite aufweist, durch die die fokussierten Ultraschallwellen entsandt werden, wobei jedes implantierbare Gehäuse mit einem halbsteifen konkaven Flügel (2₂) versehen ist, der eine Innenseite aufweist, die die Innenseite des Gehäuses verlängert und durch die auch die fokussierten Ultraschallwellen entsandt werden.

7. Behandlungssystem (1) nach einem der Ansprüche 1 bis 6, **dadurch gekennzeichnet, dass** die Ultraschallstimulationsvorrichtung (3) Mittel zur Fokussierung und variablen Orientierung der Ultraschallwellen (U), die in Richtung der Zone des angepeilten sensoriellen Kortex entsandt werden, umfasst.

8. Behandlungssystem (1) nach Anspruch 7, **dadurch gekennzeichnet, dass** die Mittel zur Fokussierung und Orientierung der Ultraschallwellen (3) elektronische Mittel zum Abtasten mindestens einer Zone des zu behandelnden sensoriellen Kortex durch die entsandten Ultraschallwellen (U) umfassen.

9. Behandlungssystem (1) nach einem der Ansprüche 1 bis 8, **dadurch gekennzeichnet, dass** die untere Wand (22) des Trägers (2) eine Außenseite umfasst, die mit einem weichen Material von variabler Dicke überzogen ist, um eine kontinuierliche Kontaktschnittstelle mit dem Gehirn (B) oder dem Dura-Mater zu bieten und die Ausbreitung der Ultraschallwellen (U) in dem Gehirn (B) zu erleichtern.

10. Behandlungssystem (1) nach einem der Ansprüche 1 bis 9, **dadurch gekennzeichnet, dass** die Regel- und Steuermittel eine externe Fernsteuerung zur drahtlosen Kontrolle und Steuerung der Ultraschallstimulationsvorrichtung umfassen.

11. Behandlungssystem (1) nach einem der Ansprüche 1 bis 10, **dadurch gekennzeichnet, dass** die Regel- und Steuermittel eine externe Steuervorrichtung umfassen, die an die Ultraschallstimulationsvorrichtung anschließbar ist.

12. Behandlungssystem (1) nach einem der Ansprüche 1 bis 11, **dadurch gekennzeichnet, dass** die elektrischen Versorgungsmittel (8) mindestens eine Speicherbatterie umfassen, die in dem Gehäuse (2) angeordnet oder subkutan am Körper des Patienten implantiert ist.

13. Behandlungssystem (1) nach einem der vorhergehenden Ansprüche, **dadurch gekennzeichnet, dass** es Elektroden (11) im Bereich einer äußeren Kontaktfläche der Innenwand (22) des Gehäuses (2) umfasst, um die elektrische Hirnaktivität zu kontrollieren und die Prävention jedes epileptischen Ereignisses zu gewährleisten.

14. Behandlungssystem (1) nach einem der vorhergehenden Ansprüche, **dadurch gekennzeichnet, dass** es einen Not-Aus-Schalter (9) in der oberen Wand des Trägers umfasst.

15. Behandlungssystem (1) nach Anspruch 1, **dadurch gekennzeichnet, dass** der elektronische Wandler (7) am Eingang mit einem System zur Bereitstellung einer sensoriellen computergestützten, in dem System gespeicherten oder von einem entfernten Gerät kommenden Information verbunden ist.

## Claims

1. A system (1) for treating at least one sensory capacity of a person comprising a stimulation device comprising an electronic converter (7) of a sensory signal into electronic signals for controlling at least one transducer for emitting signals that are images of the sensory signal, the stimulation device being a device (3) for direct ultrasound stimulation of the sensory cortex of the patient's brain (B), the ultrasound stimulation device comprising:
• at least one support (2, 2₁) that is implantable in the patient's skull (C) and that includes at least one internal wall ( **characterized in that** the ultrasound stimulation device comprises
• at least one ultrasound transducer (4) carried by the support (2) and including means for emitting focused ultrasound waves (U) through the internal wall (22) of the support without passing through the skull (C) towards a determined zone of the sensory cortex of the patient's brain (B) in order to generate modulation of the brain activity in the cortex, the ultrasound transducer (4) being driven by the electronic converter to emit focused ultrasound signals (U) that are images of the sensory signal.

2. A treatment system according to claim 1, **characterized in that** the electronic converter (7) has its input connected to at least one sensor (6, 6₁) adapted to pick up a sensory signal (5) present in the person's environment.

3. A treatment system according to claim 2, **characterized in that** the sensor (6) is a microphone adapted to pick up ambient sounds, and **in that** the ultrasound transducer (4) focuses the ultrasound waves towards a determined zone of the primary auditory cortex of the patient's brain (B).

4. A treatment system according to any one of claims 1 to 3, **characterized in that** the support comprises a unit (2) having fastener means (24) for fastening to the patient's skull and preferably in a burr hole, the unit (2) having an external wall (21) connected to the internal wall (22) via a peripheral wall (23).

5. A treatment system according to claim 2, **characterized in that** the sensor (6₁) is an image acquisition system and **in that** the ultrasound transducer (4) focuses the ultrasound waves towards a determined zone of the patient's visual cerebral cortex.

6. A treatment system according to claim 1 or claim 5, **characterized in that** the ultrasound stimulation device (3) has two implantable units (2₁) in which the ultrasound transducers (4) are mounted, each implantable unit (2₁) presenting an internal face through which the focused ultrasound waves are emitted, each implantable unit being provided with a concave semi-rigid flap (2₂) presenting an internal face extending the internal face of the unit and through which the focused ultrasound waves are also emitted.

7. A treatment system (1) according to any one of claims 1 to 6, **characterized in that** the ultrasound stimulation device (3) includes variable steering and focusing means for the ultrasound waves (U) emitted towards the target zone of the sensory cortex.

8. A treatment system (1) according to claim 7, **characterized in that** the steering and focusing means for the ultrasound waves (U) comprise means for electronically scanning at least one zone of the sensory cortex for treatment with the emitted ultrasound waves (U).

9. A treatment system (1) according to any one of claims 1 to 8, **characterized in that** the bottom wall (22) of the support (2) includes an external surface covered in a flexible material of varying thickness to provide a continuous contact interface with the brain (B) or the dura mater and to facilitate propagation of ultrasound waves (U) into the brain (B).

10. A treatment system (1) according to any one of claims 1 to 9, **characterized in that** the adjustment and control means comprise an external remote control for controlling and driving the ultrasound stimulation device wirelessly.

11. A treatment system (1) according to any one of claims 1 to 10, **characterized in that** the adjustment and control means comprise an external control device connectable to the ultrasound stimulation device (3).

12. A treatment system (1) according to any one of claims 1 to 11, **characterized in that** the electrical power supply means (8) comprise at least one rechargeable battery arranged in the unit (2) or implanted under the skin of the patient's body.

13. A treatment system (1) according to any preceding claim, **characterized in that** it includes electrodes (11) on an external contact surface of the internal wall (22) of the unit (2) for monitoring the electrical activity of the brain and for preventing any epileptic event.

14. A treatment system (1) according to any preceding claim, **characterized in that** it includes an emergency stop switch (9) in the top wall of the support.

15. A treatment system (1) according to claim 1, **characterized in that** the electronic converter (7) has its input connected to a system for delivering computerized sensory information that is stored in said system or that comes from a remote appliance.
